# EUROPEAN PATENT SPECIFICATION

(11) **EP 2 344 239 B1**
(45) Date of publication and mention of the grant of the patent: **06.11.2019**
(21) Application number: 09813942.1
(22) Date of filing: 21.09.2009
(51) Int. Cl.: A61N 1/32, A61B 5/05

(54) **DEVICE FOR REDUCING MUSCLE TENSION THROUGH ELECTRICAL MANIPULATION**
VORRICHTUNG ZUR REDUZIERUNG DER MUSKELSPANNUNG DURCH ELEKTRISCHE MANIPULATION
DISPOSITIF DE RÉDUCTION DE LA TENSION MUSCULAIRE PAR MANIPULATION ÉLECTRIQUE

(30) Priority: 19.09.2008 US 98578 P
(43) Date of publication of application: 20.07.2011
(73) Proprietor: Moore, Terry William Burton, Guelph, Ontario N1H 5J1 (CA)
(72) Inventor: Moore, Terry William Burton, Guelph, Ontario N1H 5J1 (CA)
(74) Representative: Johnstone, Douglas Ian
(86) International application number: PCT/CA2009/001318
(87) International publication number: WO 2010/031180

(56) References cited:
- US-A- 5 417 717
- US-A1- 2005 187 591
- US-A1- 2008 140 142
- US-B1- 6 212 432

## Description

### BACKGROUND OF THE INVENTION

### FIELD OF THE INVENTION

This invention relates to a device for reducing muscle tension through electrical manipulation by applying a low level current to at least one muscle of a user. More particularly, this invention relates to a device for varying the frequency and intensity of the current being applied to a muscle during specific time periods pursuant to a predetermined program that is preferably selected from a group of programs prior to the commencement of the program. Each muscle has two electrodes applied thereto, one electrode passing the current from the device to the muscle and the other electrode receiving the current from the muscle to complete the circuit. The device can also be used to diagnose an ailment of a user.

### DETAILED DESCRIPTION OF THE PRIOR ART

A method and apparatus for strengthening skeletal muscles through maximizing muscle tension in which electrical stimulation signals are applied to the selected muscles at a predetermined frequency, pulse width, and amplitude is described in Lieber, US Patent No. 4,838,272 issued on June 13, 1989. Electrical stimulation signals are applied to selected muscles at a predetermined frequency, pulse width, and amplitude to cause the muscles to contract and the work output by the muscles in response to the stimulation signals is determined over a fixed period of time. The work output is compared to a defined value, which can be a target value or a value measured during a previous stimulation period. The frequency or pulse width of stimulation signals applied to the muscles by the stimulation signals is varied in response to the results of the comparison between the work output and the defined value. The frequency is increased as the work increases and the frequency is decreased as the work decreases. The purpose of the invention described in the Lieber Patent is to increase muscle strength through long-term muscle work or activity. The output from the stimulation is transferred along the electrodes to the muscles.

The use of electrical pulses or signals to induce muscle contractions and thus stimulate muscle movement or exercise is described in the background of the Lieber U.S. Patent No. 4,838,272 to stimulate muscle movement for paralyzed limbs or for individuals having various neurological or muscular disorders to motivate non-functional muscles. Also, when injuries occur, traditional exercise may not be possible and muscles can be strengthened to decrease the impact of injuries or surgery.

It is known to reduce muscle tension through manual massage and through the use of various pharmaceuticals. Massage requires many treatments and pharmaceuticals are not very effective and not site specific. Interferential current stimulation and transcutaneous electrical nerve stimulation is known to reduce muscle tension. However, both of these forms of stimulation result in contraction of the muscle producing fatigue and the fatigued muscle has a reduced level of muscle tension. However, a fatigued muscle is electrically and biochemically different from a relaxed muscle.

The Oldham US Patent No. 6,865,423 issued March 8, 2005 describes a method for electrically stimulating a muscle and an electrical muscle stimulator. The stimulator produces a series of regularly spaced bursts of pulses with each burst, including a first component as a first continuous train of regularly spaced pulses, and second component as a series of regularly spaced second trains of regularly spaced pulses. The first component and the second component are combined and the spacing between successive pulses in the second pulse trains are less than the spacing between the successive pulses in the first pulse train. The method describes applying the stimulating signal to a muscle. The stimulation signal is applied through two electrodes, one electrode for each channel, to contract the muscle and the Patent states that it has long been established that the application of an electrical field to muscles results in an artificially induced contraction of the muscles. Each channel is coupled to one of the electrodes.

Patent US 6,212,432 B1 discloses an oscillating unit for providing therapeutic electrical currents supplied via electrodes connectable to a patient. The oscillating unit generates plural frequencies chosen from frequencies determined in advance and delivers them from the lowest frequency in an ascending order at specified time intervals.

### SUMMARY OF THE INVENTION

The invention is defined in claim 1. Any embodiment which is in contradiction to the subject-matter of claim 1 is not part of the invention.

It is an object of the present invention to provide a device of applying a low level alternating current to muscles of a user, using a device having a controller to control a voltage of the device and a frequency and intensity of the current applied to one or more muscles of the user at a series of predetermined frequencies for predetermined times for each frequency. It is a further object of the present invention to have a controller in the device where the controller can be programmed and predetermined in advance for each user to apply a current to one or muscles through electrodes by a series of predetermined frequencies for predetermined times for each frequency. Preferably, the device has a plurality of circuits with a separate circuit being used for each muscle. Each circuit preferably has two electrodes, one of the two electrodes for passing current to a muscle and the other of two electrodes for receiving current from the muscle to complete the circuit. It is a further object of the present invention to provide a device for causing one or more muscles of a user to relax through electrical manipulation at a level below the threshold level that would cause the one or more muscles to contract.

It is a further object of the invention to provide a device that results in muscle relaxation through electrical manipulation at an intensity level below the threshold level at which a muscle will contract without producing muscle fatigue.

A method of relaxing muscle tension through electrical manipulation by applying a low level alternating current to muscles of a user is provided. The method uses a device having a power source and a controller to control a voltage and frequency of the alternating current applied to at least one muscle for a given period of time. The device has a plurality of electrodes and is programmed with at least one program. The method comprises connecting the device to a power source, connecting two electrodes of said plurality of electrodes to each muscle of said at least one muscle, activating the program for the device to apply to the at least one muscle of the user a low level alternating current at a series of predetermined frequencies in succession for predetermined times for each frequency, at a predetermined intensity of current for each frequency, applying two electrodes of the plurality of electrodes to each muscle of the at least one muscle, activating the device to carry out the program by passing the current through each muscle of the at least one muscle from one of the two electrodes of each muscle to the other of the two electrodes of each muscle, deactivating the device and removing the electrodes upon completion of the program.

A device for reducing muscle tension through electrical stimulation by applying a low level alternating current to at least one muscle of a user is provided. The device comprises a power source, a plurality of electrodes, two electrodes of the plurality of electrodes being in contact with each muscle of the at least one muscle of the user. One electrode on each muscle is connected to supply current to the at least one muscle, and another electrode on each muscle is connected to receive current from the at least one muscle to complete the circuit from the device for each muscle. The electrodes on each muscle constitute a pair of electrodes. A controller controls a frequency and intensity of the alternating current for fixed periods of time and automatically varies the frequency and time for which a particular frequency and intensity of current is applied to the at least one muscle. The intensity is adjustable by the controller prior to commencing a program of the device, the program applying the current at predetermined frequencies for predetermined times in succession. The program is set for each user in advance of activating the program for that user. The intensity is capable of being set below a threshold of causing each muscle of the at least one muscle to contract.

A method of diagnosing ailments through electromanipulation by applying a low level alternating current to muscles of a user is provided. The method uses a device having a power source and a controller to control a voltage and frequency of the alternating current applied to at least one muscle for a given period of time. The device has a plurality of electrodes and the device is programmed. The method comprises connecting the device to the power source, connecting two of the plurality of electrodes to each muscle of said at least one muscle, activating the program for the device to apply to the at least one muscle a low level alternating current at a first frequency for a pre-determined time, using the controller to varying intensity of the low level current to each muscle of the at least one muscle to a level intensity that is slightly lower than a level required to cause the at least one muscle to contract or twitch and determining a voltage of the device at the level of intensity of the current for each muscle of the at least one muscle, using the voltage to diagnose the ailment of user by comparing the voltage to a range of voltages for normal muscles and determining whether the voltage is higher or lower than the range.

### BRIEF DESCRIPTION OF THE DRAWINGS

Figure 1 is a block diagram showing the manner in which various components of the device are connected; and
Figure 2 is a perspective view of a front of a device for applying low level current.

### DETAILED DESCRIPTION OF A PREFERRED EMBODIMENT

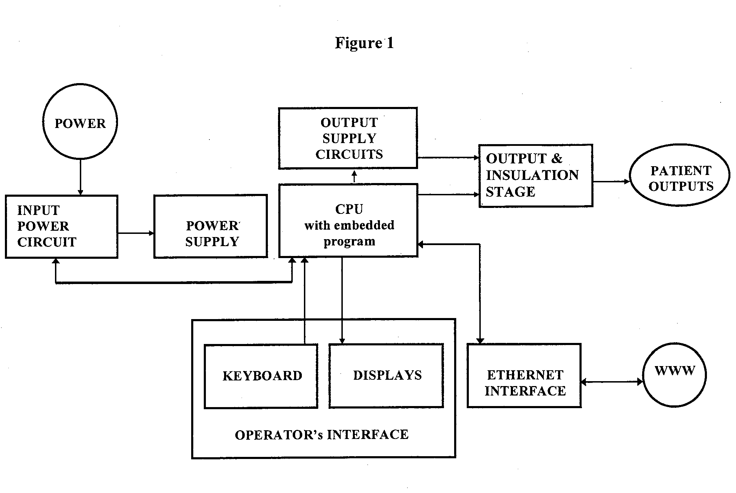

In Figure 1, in a block diagram, it is shown that a device for applying a low level current to at least one muscle of a user has an input power circuit which is connected to a power supply with an on/off switch that is designated power. The input power circuit is connected to a central processing unit ("CPU") with an embedded software program. The CPU is connected to output supply circuits and to an output and insulation stage. The output supply circuits are also connected to the output and insulation stage in parallel to the CPU. The output and insulation stage is connected to patient outputs or electrodes. The CPU is also connected to a keyboard, which enables an operator to input information or vary the parameters and to displays which display the information that has been inputted and the progress of the process after it has been initiated. The CPU can also be connected to an ethernet interface which allows access to the world wide web to allow remote monitoring and/or control of the device.

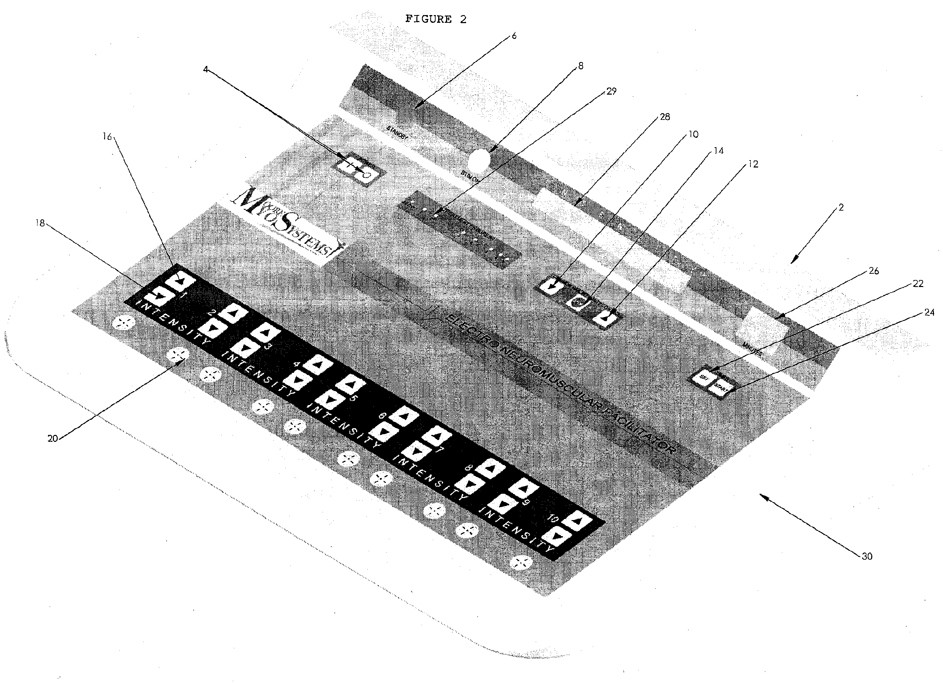

In Figure 2, there is shown a front view of a device 2 for applying low level current to muscles of a patient user. A power cord for connecting the device to an AC power source is not shown in Figure 2 and is conventional. The device has an on/off switch 4 with a standby indicator lamp 6 and stimulator indicator lamp 8. There are intensity control buttons 10, 12 for decreasing or increasing respectively the current intensity to electrodes (not shown in Figure 2). An indicator light 14 is located between the intensity control buttons 10, 12. Near the front of the device 2 are a series of intensity control buttons 16, 18 where the buttons 16 are depressed to increase intensity and the buttons 18 are depressed to decrease intensity. It will be noticed that each pair of buttons 16, 18 from left to right is numbered from 1 to 10 and represents a channel as there are ten pairs of buttons 16, 18. There is also a number of output sockets 20, with one socket being available for each pair of intensity control buttons 16, 18. A double wire (not shown) for each pair of electrodes (not shown) has a plug (not shown) at one end and two electrodes at the other end. One of the two wires is connected to each electrode and the plug is compatible with the sockets 20. On the right hand side of the device 2, there are timer setting buttons 22, 24 and a timer display 26. At the approximate center near the back of the device 2, there is a parameter and program display 28, which displays the inputs entered into the device for each patient user. Between the on/off switch 4 and the intensity control buttons 10, 12, there are located treatment progress indicator lamps 29 to show the progress of the program being carried out with respect to a patient. On the right hand side of the device 2, there is an ethernet connector socket 30.

Preferably, the device is programmed to apply the frequencies in 20 second bursts, with 0.5 seconds between bursts. There are preferably 20 second trains of electrical impulses at a particular frequency with a 0.5 second gap between each train. Preferably, the pulse width is of 200 microsecond duration and the wave form is rectangular in nature. The maximum voltage is preferably 70 volts with a peak at a resistance of 1 K ohm. Each train represents one frequency and trains are successive.

In a method of operating the device, the power switch is turned on and the standby indicator light is illuminated. The electrodes, which have pads (not shown) are placed on the user with one electrode being placed at either end of the muscle to be treated. The user can more particularly be described as a patient. The electrodes are connected through the double wire assembly, as described above, to an output socket. When the device is removed from the standby mode and activated, and the electrodes (not shown) are in contact with a user, current flows from the device to a first electrode at one end of the muscle to be treated to a second electrode at the other end of the muscle to be treated and back to the device to complete the circuit. The electrode has conductive material located between the electrodes and the patient's body. This provides a buffer for the current to be applied to the muscle. The device can be used to treat more than one muscle at a time, and preferably when the muscle or muscles being treated are on one side of a patient's body, the same muscle or muscles on the other side of the patient will also be treated. For example, if the muscle on the right hand side of the patient is to be treated, the same muscle on the left hand side of the patient will be treated simultaneously even if the muscle on the left hand side is not causing any discomfort to the patient. The most important muscles to be treated on the back of a patient are located 1-2 cm lateral to the spinal processes.

After all the electrodes are secured to the patient, using straps, adhesive surfaces, suction cups or other means of securing the electrodes, the patient will move to a comfortable position, and will usually be asked to lie on the electrodes. If the electrodes are located on the patient's back, the patient will turn over after the electrodes have been placed, and lie on his/her back.

The operator of the device then chooses and sets the treatment time, preferably for substantially 30, 45, 60 or 90 minutes, as is deemed appropriate for a particular patient. When the treatment time has been selected, an operator then begins to set the intensity of the current for each of the electrode channels, there being one electrode channel for each muscle being treated. Each electrode channel is a separate circuit for the muscle that is being treated, each channel having two electrodes. The current applied by the device to a particular muscle flows from the device through one of the electrodes, through the muscle of the patient or user, and back to the device through the other electrode. It is important to set the intensity settings to a level just below the threshold for a muscle twitch or contraction for the muscle that is being treated. The operator increases the intensity of the current for the electrodes on the particular muscle to produce a mild twitch or mild contraction in the muscle and then slowly decreases the intensity of the current until only the sensation of the current is perceived by the patient, but there is no twitch or contraction feeling or any appearance of a twitch or contraction in the muscle. The contraction can be a tetanic or twitch contraction. Each of the electrodes is set to the same level of intensity for a particular muscle and the level of intensity is set for each of the muscles to be treated during the same treatment time below the threshold for each muscle that causes a contraction. For muscles that correspond to one another, the appropriate intensity level can be determined for one of the muscles, but must still be independently determined for the other corresponding muscle. The corresponding muscle is a muscle on one side of the body that corresponds to the same muscle on the other side of the body of the patient. When all of the electrodes are in place and the level of intensity has been established the operator proceeds to start the treatment by pressing the start button 24, which is the stimulation on lamp in Figure 2.

At this time, the operator may record the percentage of intensity of each channel of the device for comparison to normative values and/or comparison to past or future intensity settings for that patient.

The program of the device will proceed through different frequencies at their proper time allotments automatically based upon the total program time chosen by the operator for that patient and inputted it into the device as stated above. The sequence of frequencies are predetermined and preferably pre-programmed into the device through a programmable controller. The preferred frequencies and the sequence thereof for a total treatment time of 30 minutes is 80 hertz for 15 minutes, 4 hertz for 4 minutes, 30 hertz for 5 minutes, 50 hertz for 2 minutes, 80 hertz for 2 minutes and 120 hertz for 2 minutes. Still more preferably, the frequencies and the sequence thereof for a total treatment time of 30 minutes is 80 hertz for 5 minutes, 120 hertz for 10 minutes, 4 hertz for 4 minutes, 30 hertz for 5 minutes, 50 hertz for 2 minutes, 80 hertz for 2 minutes and 120 hertz for 2 minutes. The times are preferred times, but other times are suitable as well.

When the treatment program is commenced, the operator should instruct the patient to signal the operator if they feel any pain or discomfort, or twitch or contraction in their muscles. The operator should be able to audibly monitor the patient during treatment and, if necessary, make adjustments to the intensity of the electrodes to ensure proper intensity levels are maintained throughout the treatment. In most cases, no adjustment will be required after the intensity level is set for each muscle before the program commences. When the treatment is completed, the machine will produce an audio signal and automatically shut down. The electrode pads are removed from the patient and arrangements are made for a subsequent treatment or treatments, if required. Most often, several treatments are required and the number of treatments usually ranges from 2 to 5, but each case must be considered independently and the number of treatments can vary widely. The sequence of frequencies and times set out above is for a 30 minute treatment program. When the treatment program is longer than 30 minutes, the times for each frequency increase proportionately. The factor is 1.5, 2 and 3 for 45 minutes, 60 minutes and 90 minutes respectively. When the treatment time is increased beyond thirty minutes, the device automatically increases the time at each frequency proportionally. While the exact frequencies and times set out in the sequence are preferred, substantially the same frequencies and times can also be used. A range of frequencies for the 30, 50, 80 and 120 hertz is ± 5 hertz and the range for the 4 hertz frequency is ± 2 hertz. The preferred ranges of frequency, in order of application are therefore 75 to 85 hertz, 115 to 125 hertz, 2 to 6 hertz, 25 to 35 hertz, 45 to 55 hertz, 75 to 85 hertz and 115 to 125 hertz. The treatment times are flexible and the time at each frequency is also flexible. Electrical impulses at 50 hertz and 80 hertz decrease the release of Leukotriene B4, a potent inflammatory substance. It is known that a muscle of shortened length cannot use the optimal compliment of acto-myosin bridges. The muscle therefore cannot generate as much muscle tension. A shortened muscle (caused by muscle tightness/spasm) does not regain full strength or endurance regardless of the strength and endurance exercises. A muscle in spasm or excessively tight, results in the release of several inflammatory compounds, including Leukotriene B4, due to the hypoxia that results from physical compression of the capillary beds. However, Leukotriene B4 reduces the blood flow in the muscle, resulting in the muscle fatiguing and tightening further, perpetuating the cycle. This perpetuating cycle of muscles fatiguing and tightening further can in some cases compress lymphatic vessels, causing an increase of fluid in the lymph vessel and the interstitial fluid also builds up. The result can be substantial swelling. This swelling can further compress the blood vessels causing the release of more Leukotriene B4 and this becomes a vicious circle. With the device of the present invention, muscles can be made to relax, without causing the muscles to contract during the program. The purpose of the device of the present invention is not to work the muscle(s), but to relax the muscle(s) of a user.

While the device of the present invention are particularly suited for lower back pain with the electrodes being in contact on either side of the back of the patient, the device can also be used to treat muscles and other parts of the body, for example, the arms and legs of a patient. For example, the device of the present invention can be used in the following therapeutic applications or ailments which are not intended to be exhaustive:
Muscle spasms (motor vehicle accident, occupational, personal injuries, stress)
Repetitive strain injuries
   Carpal tunnel syndrome epicondylitis
   Bursitis,
   Tendonitis,
Headaches
   Muscular,
   Vascular,
Radiculopathies
Frozen shoulder/thoracic outlet syndrome
Trigeminal Neuralgia
Temporal-Mandibular Joint Pain Multiple Sclerosis
Parkinson's Disease
Stroke Survivors
Disc Injury or Degeneration
Chronic Fatigue Syndrome
Arthritis
   Osteoarthritis
   Rheumatoid
   Arteritis
Polymyalgia
Fibromyalgia
Lupus

The device of the present invention are effective in eliminating or reducing pain caused by injury or disease and is also effective in preventing further injury. The system results in relaxing the muscles treated. The process of the present invention relates generally to electrical manipulation of muscles for diagnostic purposes, rehabilitation therapy as well as treatment of specific diseases, conditions and injuries. The process of the present invention reduces the tension in specific muscles alleviating symptoms of the muscles to which it is applied. The process will allow for any nerve root compressions (when applied to paraspinal muscles), joint, vessel or other structures compressed by muscles to be released mitigating these results and dysfunctions, paraesthesias and/or pain symptoms. The invention can be used diagnostically as well as therapeutically.

For diagnostics concerning the skeletal muscles, in the first step of the method after the electrodes have been connected to apply low level alternating current to said at least one muscle in the first frequency range of 75-85 hertz and, preferably, at a frequency of 80 hertz, the device of the present invention can be used to determine the voltage that results in the device at that frequency (or range of frequencies) when the intensity of the current is set at a level that is slightly lower than a level required to cause the at least one muscle to contract or twitch. The other variables are the same as the initial settings for the therapeutic approach. At a frequency of 80 hertz, there is a narrow band of voltages that results in a maximum sub-threshold for contraction in healthy muscle tissue. This band of voltages ranges from 1.6 to 1.8 volts.

Voltage that is below the minimum voltage in the above range indicates a muscle that is hypersensitive as well as in spasm. Voltage that is higher than the maximum voltage in the above range indicates a muscle that is in very severe spasm. If the minimum threshold levels fall within the normal range, the muscle tissue is healthy and at most may require strength and endurance building. Therefore, the device of the present invention can be used to diagnose the location and the status of muscular problems that underlie various ailments including back pain, repetitive strain injuries, Multiple Sclerosis symptoms, Parkinson's Disease symptoms and Fibromyalgia among various other conditions.

The device increases the intensity level by increasing the current and the current is increased by increasing the voltage of the particular channel to which the electrodes are connected. Preferably, there is no overlap of frequencies and the frequencies of current are applied in succession.

### Example 1

Electromanipulation of muscles for the treatment of repetitive strain injury: A Pilot study.

### Aims:

The objective was to identify the efficacy of the protocol including the device of the present invention for patients suffering from pain in the forearm, wrist and hand caused by repetitive strain injury (RSI).

### Method:

Seventeen volunteers participated in the study. Twelve females (mean age 41.5 plus or minus 9.9) and five males (mean age 35 plus or minus 8.7), followed the protocol for a minimum of six and a maximum of thirteen sessions (9.7 plus or minus 1.4) within one month. Subjects were excluded from this study if they presented with severe obesity (BMI >34.9) and/or pregnancy/ All subjects followed the same protocol: electromyotherapy using the device of the present invention on specific muscles followed by a ten minute trigger point release and a protocol of stretches and strengthening for the neck and upper back muscles. The Board pain scale was used to acquire pain levels. Grip strength was measured using a hand dynamometer.

### Results:

Pain levels were gathered before and after each session. For the purpose of this study, only data from the first (PTxI) and the last visit (PTxlas) was analyzed. Grip strength was measured prior to the first treatment (GTxI) and after the last visit (GTxlas). All data was analyzed using the One-way ANOVA and TUKEY' S post hoc comparisons. Results were statistically significant (p< 0.05) for PTxI and (p=0.00) for PTxlas. No significant differences was found for GTxI and GTxlas (p=0.12).

### Conclusion:

Preliminary data shows that perceived pain levels decreased after treatment following the above protocol for one month. This improvement leads to the conclusion that the protocol can be an efficient tool in the treatment of RSI.

### Example 2

Electromanipulation of muscles for the treatment of low back pain.

### Aims:

The objective was to identify the efficiency of the protocol including the device of the present invention for patients suffering from low back pain (LBP).

### Method:

Fifteen volunteers participated in the study. Eight females (mean age 47.5 plus or minus 12.4) and seven males (mean age 41.8 plus or minus 13.0), followed the protocol for a minimum of four and maximum often sessions within two months. Volunteers were excluded from this study if they presented with neurological deficits in the legs due to a medical condition other than the back, severe obesity (BMI >34.9), severe spinal stenosis, Fibromyalgia and/or pregnancy. All subjects followed the same protocol: electromyo therapy using the device of the present invention on specific muscles followed by a ten minute trigger point release and a protocol of stretches and strengthening for the low back muscles. The Borg pain scale was used to acquire pain levels.

### Results:

Pain levels were gathered prior to and after the first treatment (TxI), fourth session (Tx4) and last visit (Txlas). All data was analyzed using One-way ANOVA. Results were statistically significant (p<0.05) when comparing pre and post for TxI (p=0.03) and Txlas (p=0.02). A significant difference was also found when comparing TxI to Tx4 (p=0.00), Tx4 to Txlas (p=0.00) and TxI to Txlas (p=0.00).

### Conclusion:

Perceived pain levels were lower after subjects received their first treatment with the electrical manipulation in accordance with the present invention and significantly decreased after completion of the treatment plan. This improvement leads us to believe that the above protocol is an efficient tool in the treatment of LBP.

### Example 3

A physical therapy approach in the treatment of Multiple Sclerosis.

### Aim:

To determine the efficacy of the protocol including the device of the present invention for patients suffering from MS of various types and durations.

### Method:

Thirteen volunteers participated in this study, nine females (mean age 42.1 plus or minus 11.9) and four males (mean age 43.3 plus or minus 3.3). Volunteers were excluded if they presented with severe obesity (BMI>34.9), Fibromyalgia, severe spinal stenosis or pregnancy. All subjects followed the same protocol: electromyotherapy using the device of the present invention on specific muscles followed by a ten minute trigger point release and a program of stretching and strengthening the specific muscles. The participants received four to thirteen treatments within seven weeks. The Multiple Sclerosis Impact Scale (MSIS-29) was used to determine the degree of dysfunction at intake and completion. All data was analyzed using a One-way ANOVA.

### Results:

All participant scores improved. The mean improvement was 25.6 plus or minus 18.4%. Participants demonstrated levels of individual improvement from 1.6% to 60.7% at completion. The results were statistically significant (p=0.03).

### Conclusion:

The above protocol can be an effective physical therapeutic approach for treating MS of various types and durations.

## Claims

1. A device (2) for reducing muscle tension through electrical manipulation by applying a low level alternating current to at least one muscle of a user, said device comprising a power source, a plurality of electrodes, two electrodes of said plurality of electrodes being in contact with each muscle that is being treated of said at least one muscle of the user, one electrode on each muscle that is being treated being connected to supply current to that muscle and another electrode on that muscle being connected to receive current from that muscle to complete a circuit from said device for each muscle being treated, there being a separate circuit for each muscle being treated, said two electrodes on each muscle constituting a pair of electrodes, a controller to control a frequency and intensity of said alternating current for fixed periods of time and to automatically vary a frequency and time for which a particular frequency and intensity of the current is applied for each circuit, said intensity being settable for each circuit by an operator below a threshold of causing each muscle being treated of said at least one muscle to contract prior to commencing a program of said device, said program causing said device to apply said current at predetermined frequencies for predetermined times in succession for each circuit, said program being set for each user in advance of activating said program for that user.

2. A device as claimed in Claim 1 wherein said controller is programmable and said device is capable of applying automatically at least six different frequencies in succession for six different time periods for each circuit, there being one frequency for each time period.

3. A device as claimed in Claim 1 or 2 wherein said controller is programmable and said device is capable of applying seven different frequencies automatically for seven different time periods for each circuit.

4. A device as claimed in Claim 2 wherein said frequencies and time periods for each circuit are: substantially 80 hertz, 4 hertz, 30 hertz, 50 hertz, 80 hertz, and 120 hertz for times of substantially 15 minutes, 4 minutes, 5 minutes, 2 minutes, 2 minutes and 2 minutes respectively for a total of substantially 30 minutes.

5. A device as claimed in Claim 3 wherein said frequencies and time periods for each circuit are: substantially 80 hertz, 120 hertz, 4 hertz, 30 hertz, 50 hertz, 80 hertz and 120 hertz for times of substantially 5 minutes, 10 minutes, 4 minutes, 5 minutes, 2 minutes, 2 minutes and 2 minutes respectively for a total of 30 minutes.

6. A device as claimed in Claim 4 wherein said device is capable on increasing said time periods for each frequency by a factor selected from the group of 1.5, 2 and 3 resulting in an increased total time.

7. A device as claimed in Claim 5 wherein said device is capable of increasing said time periods for each frequency by a factor selected from the group of 1.5, 2 and 3 resulting in an increased total time.

8. A device as claimed in any preceding Claim wherein said current has a waveform that is rectangular and said current is applied from said device in 20 second bursts with 0.5 seconds between each burst.

9. A device as claimed in any preceding Claim wherein said total time period is substantially 30 minutes.

10. A device as claimed in Claim 1 wherein said frequencies are substantially constant within a range of frequencies for each circuit, and said time periods are within 75-85 hertz, 2-6 hertz, 25-35 hertz, 45-55 hertz, 75-85 hertz, and 115-125 hertz, for times of substantially 15 minutes, 4 minutes, 5 minutes, 2 minutes, 2 minutes, and 2 minutes, respectively, for a total time of substantially 30 minutes.

11. A device as claimed in Claim 1, wherein said frequencies are substantially constant frequencies within a range of frequencies for each circuit, and said time periods are within 75-85 hertz, 115-125 hertz, 2-6 hertz, 25-35 hertz, 45-55 hertz, 75-85 hertz, and 115-125 hertz, for times of substantially 5 minutes, 10 minutes, 4 minutes, 5 minutes, 2 minutes, 2 minutes, and 2 minutes, respectively, for a total time of substantially 30 minutes.

12. A device as claimed in any preceding Claim wherein said device has a plurality of channels with each pair of electrodes being connected to a different channel, said controller being constructed to control said intensity of current from each channel separately.

13. A device as claimed in any preceding Claim wherein said device has a plurality of programs for applying said current to one or more muscles of said user, said programs having different total times and said device being operable to run only one of said programs at any given time.

## Patentansprüche

1. Eine Vorrichtung (2) zur Reduzierung von Muskelspannung durch elektrische Manipulation durch Anlegen eines niedrigen Wechselstroms an zumindest einen Muskel eines Anwenders, wobei die besagte Vorrichtung eine Stromquelle umfasst, eine Vielzahl von Elektroden, wobei zwei Elektroden der besagten Vielzahl an Elektroden in Kontakt mit jedem Muskel stehen, der von dem besagtem, zumindest einen Muskel des Anwenders behandelt wird, wobei eine Elektrode an jedem Muskel, der behandelt wird verbunden ist, um Strom an jenen Muskel zu liefern, und eine weitere Elektrode an jenem Muskel verbunden ist, um Strom von dem Muskel zu erhalten, um einen Stromkreis der Vorrichtung für jeden zu behandelnden Muskel zu schließen, wobei es für jeden Muskel, der behandelt wird einen separaten Stromkreis gibt, wobei die besagten zwei Elektroden an jedem Muskel ein Paar Elektroden bilden, wobei eine Regelung zur Steuerung einer Frequenz und der Intensität des besagten Wechselstroms für festgelegte Zeiträume und zum automatischen Verändern einer Frequenz und einer Zeit, für die eine bestimmte Frequenz und Intensität des Stroms für jeden Stromkreis angelegt wurde, vorgesehen ist, wobei die besagte Intensität für jeden Stromkreis von einer Bedienperson unterhalb eines Grenzwertes einstellbar ist, was dazu führt, dass jeder Muskel von dem besagten, zumindest einen Muskel, der behandelt wird, kontrahiert, bevor ein Programm der besagten Vorrichtung einsetzt, wobei das besagte Programm dazu führt, dass die besagte Vorrichtung den besagten Strom in vorgegebenen Frequenzen zu vorgegebenen Zeiten nacheinander für jeden Stromkreis anlegt, wobei das besagte Programm für jeden Benutzer vor der Aktivierung des besagten Programms für jenen Benutzer eingestellt wird.

2. Eine Vorrichtung gemäß Anspruch 1, wobei die besagte Regelung programmierbar und die besagte Vorrichtung in der Lage ist, zumindest sechs verschiedene Frequenzen für sechs verschiedene Zeiträume für jeden Stromkreis nacheinander automatisch anzulegen, wobei es eine Frequenz für jeden Zeitraum gibt.

3. Eine Vorrichtung gemäß Anspruch 1 oder 2, wobei die besagte Regelung programmierbar und die besagte Vorrichtung in der Lage ist, sieben verschiedene Frequenzen für sieben verschiedene Zeiträume für jeden Stromkreis automatisch anzulegen.

4. Eine Vorrichtung gemäß Anspruch 2, wobei die besagten Frequenzen und Zeiträume für jeden Stromkreis lauten: im Wesentlichen 80 Hertz, 4 Hertz, 30 Hertz, 50 Hertz, 80 Hertz und 120 Hertz für Zeiträume von im Wesentlichen 15 Minuten, 4 Minuten, 5 Minuten, 2 Minuten, 2 Minuten und 2 Minuten entsprechend für eine Gesamtzeit von im Wesentlichen 30 Minuten.

5. Eine Vorrichtung gemäß Anspruch 3, wobei die besagten Frequenzen und Zeiträume für jeden Stromkreis lauten: im Wesentlichen 80 Hertz, 120 Hertz, 4 Hertz, 30 Hertz, 50 Hertz, 80 Hertz und 120 Hertz für Zeiträume von im Wesentlichen 5 Minuten, 10 Minuten, 4 Minuten, 5 Minuten, 2 Minuten, 2 Minuten und 2 Minuten entsprechend für eine Gesamtzeit von 30 Minuten.

6. Eine Vorrichtung gemäß Anspruch 4, wobei die besagte Vorrichtung in der Lage ist, die besagten Zeiträume für jede Frequenz um einen Faktor, der aus der Gruppe von 1.5, 2 und 3 ausgewählt wurde, zu erhöhen, was zu einer erhöhten Gesamtzeit führt.

7. Eine Vorrichtung gemäß Anspruch 5, wobei die besagte Vorrichtung in der Lage ist, die besagten Zeiträume für jede Frequenz um einen Faktor, der aus der Gruppe von 1.5, 2 und 3 ausgewählt wurde, zu erhöhen, was zu einer erhöhten Gesamtzeit führt.

8. Eine Vorrichtung gemäß irgendeinem der vorangehenden Ansprüche, wobei der besagte Strom eine Wellenform aufweist, die rechteckig ist, und der besagte Strom wird von der besagten Vorrichtung in Stößen von 20 Sekunden mit einer Unterbrechung von 0,5 Sekunden zwischen jedem Stoß angelegt.

9. Eine Vorrichtung gemäß irgendeinem der vorangehenden Ansprüche, wobei die besagte Gesamtzeit im Wesentlichen 30 Minuten beträgt.

10. Eine Vorrichtung gemäß Anspruch 1, wobei die besagten Frequenzen innerhalb eines Bereiches von Frequenzen für jeden Stromkreis im Wesentlichen konstant sind, und die besagten Zeiträumen liegen innerhalb von 75-85 Hertz, 2-6 Hertz, 25-35 Hertz, 45-55 Hertz, 75-85 Hertz und 115-125 Hertz für Zeiträume von im Wesentlichen 15 Minuten, 4 Minuten, 5 Minuten, 2 Minuten, 2 Minuten und 2 Minuten entsprechend einer Gesamtzeit von im Wesentlichen 30 Minuten.

11. Eine Vorrichtung gemäß Anspruch 1, wobei die besagten Frequenzen im Wesentlichen konstante Frequenzen innerhalb eines Bereiches von Frequenzen für jeden Stromkreis sind, und die besagten Zeiträume liegen innerhalb von 75-85 Hertz, 115-125 Hertz, 2-6 Hertz, 25-35 Hertz, 45-55 Hertz, 75-85 Hertz und 115-125 Hertz für Zeiträume von im Wesentlichen 5 Minuten, 10 Minuten, 4 Minuten, 5 Minuten, 2 Minuten, 2 Minuten und 2 Minuten entsprechend einer Gesamtzeit von im Wesentlichen 30 Minuten.

12. Eine Vorrichtung gemäß irgendeinem der vorangehenden Ansprüche, wobei die besagte Vorrichtung eine Vielzahl von Kanälen aufweist, wobei jedes Paar Elektroden mit einem anderen Kanal verbunden ist, wobei die besagte Regelung so konstruiert ist, um die besagte Intensität des Stroms von jedem Kanal separat zu steuern.

13. Eine Vorrichtung gemäß irgendeinem der vorangehenden Ansprüche, wobei die besagte Vorrichtung eine Vielzahl von Programmen aufweist, um den besagten Strom an einen oder mehrere Muskel des besagten Anwenders anzulegen, wobei die besagten Programme verschiedene Gesamtzeiten aufweisen und die besagte Vorrichtung so bedient werden kann, dass nur eines der besagten Programme zu irgendeiner vorgegebenen Zeit läuft.

## Revendications

1. Dispositif (2) destiné à réduire la tension musculaire par manipulation électrique, en appliquant un courant alternatif de faible niveau à au moins un muscle d'utilisateur, ledit dispositif comprenant une source d'alimentation, une pluralité d'électrodes, deux électrodes de ladite pluralité d'électrodes étant en contact avec chaque muscle qui est en cours de traitement dudit au moins un muscle de l'utilisateur, une électrode sur chaque muscle qui est en cours de traitement étant connectée pour fournir un courant à ce muscle et une autre électrode sur ce muscle étant connectée pour recevoir un courant en provenance de ce muscle afin de fermer un circuit à partir dudit dispositif pour chaque muscle en cours de traitement, un circuit séparé existant pour chaque muscle en cours de traitement, lesdites deux électrodes sur chaque muscle constituant une paire d'électrodes, un dispositif de commande pour commander une fréquence et une intensité dudit courant alternatif pendant des périodes de temps fixes et pour faire varier automatiquement une fréquence et une durée pendant laquelle une fréquence et une intensité particulières du courant sont appliquées pour chaque circuit, ladite intensité étant réglable pour chaque circuit par un opérateur, au-dessous d'un seuil faisant se contracter chaque muscle en cours de traitement dudit au moins un muscle, avant le début d'un programme dudit dispositif, ledit programme faisant appliquer audit dispositif ledit courant à des fréquences prédéterminées pendant des durées prédéterminées, successivement pour chaque circuit, ledit programme étant défini pour chaque utilisateur préalablement à l'activation dudit programme pour cet utilisateur.

2. Dispositif selon la revendication 1, dans lequel ledit dispositif de commande est programmable et ledit dispositif est apte à appliquer automatiquement au moins six fréquences différentes, successives, pendant six périodes de temps différentes pour chaque circuit, une fréquence existant pour chaque période de temps.

3. Dispositif selon la revendication 1 ou 2, dans lequel ledit dispositif de commande est programmable et ledit dispositif est apte à appliquer automatiquement sept fréquences différentes, pendant sept périodes de temps différentes pour chaque circuit.

4. Dispositif selon la revendication 2, dans lequel lesdites fréquences et périodes de temps pour chaque circuit sont : sensiblement 80 hertz, 4 hertz, 30 hertz, 50 hertz, 80 hertz et 120 hertz pendant des durées de sensiblement 15 minutes, 4 minutes, 5 minutes, 2 minutes, 2 minutes et 2 minutes respectivement pour un total de sensiblement 30 minutes.

5. Dispositif selon la revendication 3, dans lequel lesdites fréquences et périodes de temps pour chaque circuit sont : sensiblement 80 hertz, 120 hertz, 4 hertz, 30 hertz, 50 hertz, 80 hertz et 120 hertz pendant des durées de sensiblement 5 minutes, 10 minutes, 4 minutes, 5 minutes, 2 minutes, 2 minutes et 2 minutes respectivement pour un total de 30 minutes.

6. Dispositif selon la revendication 4, dans lequel ledit dispositif est apte à accroître lesdites périodes de temps pour chaque fréquence, d'un facteur choisi dans le groupe de 1,5 ; 2 et 3 donnant lieu à une durée totale accrue.

7. Dispositif selon la revendication 5, dans lequel ledit dispositif est apte à accroître lesdites périodes de temps pour chaque fréquence, d'un facteur choisi dans le groupe de 1,5 ; 2 et 3 donnant lieu à une durée totale accrue.

8. Dispositif selon l'une quelconque des revendications précédentes, dans lequel chaque courant a une forme d'onde qui est rectangulaire et ledit courant est appliqué depuis ledit dispositif par salves de 20 secondes avec 0,5 seconde entre chaque salve.

9. Dispositif selon l'une quelconque des revendications précédentes, dans lequel ladite période de temps totale est sensiblement de 30 minutes.

10. Dispositif selon la revendication 1, dans lequel lesdites fréquences sont sensiblement constantes à l'intérieur d'une gamme de fréquences pour chaque circuit, et lesdites périodes de temps se situent entre 75 et 85 hertz, 2 et 6 hertz, 25 et 35 hertz, 45 et 55 hertz, 75 et 85 hertz et entre 115 et 125 hertz, pendant des durées de sensiblement 15 minutes, 4 minutes, 5 minutes, 2 minutes, 2 minutes et 2 minutes, respectivement, pour une durée totale de sensiblement 30 minutes.

11. Dispositif selon la revendication 1, dans lequel lesdites fréquences sont des fréquences sensiblement constantes à l'intérieur d'une gamme de fréquences pour chaque circuit, et lesdites périodes de temps se situent entre 75 et 85 hertz, 115 et 125 hertz, 2 et 6 hertz, 25 et 35 hertz, 45 et 55 hertz, 75 et 85 hertz et entre 115 et 125 hertz, pendant des durées de sensiblement 5 minutes, 10 minutes, 4 minutes, 5 minutes, 2 minutes, 2 minutes et 2 minutes, respectivement, pour une durée totale de sensiblement 30 minutes.

12. Dispositif selon l'une quelconque des revendications précédentes, dans lequel ledit dispositif possède une pluralité de canaux, chaque paire d'électrodes étant connectée à un canal différent, chaque dispositif de commande étant conçu pour commander ladite intensité de courant à partir de chaque canal séparément.

13. Dispositif selon l'une quelconque des revendications précédentes, dans lequel ledit dispositif comporte une pluralité de programmes destinés à appliquer ledit courant à un ou plusieurs muscle(s) dudit utilisateur, lesdits programmes ayant des durées totales différentes et ledit dispositif permettant de n'exécuter qu'un seul desdits programmes à un moment donné.
